# EUROPEAN PATENT SPECIFICATION

(11) **EP 1 344 452 B1**
(45) Date of publication and mention of the grant of the patent: **30.11.2005**
(21) Application number: 02005881.4
(22) Date of filing: 14.03.2002
(51) Int. Cl.: A01K 45/00, C12M 3/00

(54) **Method for shell-less in vitro culture of avian embryos**
Verfahren zur Retortenaufzucht von Geflügelembryonen
Procédé de culture d'embryons de volaille in vitro

(43) Date of publication of application: 17.09.2003
(73) Proprietor: NAMAXX GENOMICS GMBH, 70569 Stuttgart (DE)
(72) Inventor: Dr. Villatte, Francois, 70569 Stuttgart (DE); Dr. Bachmann, Till T., 70569 Stuttgart (DE)
(74) Representative: Becker Kurig Straus

(56) References cited:
- DE-C- 586 375

## Description

The present invention relates to a method for shell-less in vitro culture of avian embryos. In particular, the present invention is concerned with the use of a device for a shell less in vitro culture of avian embryos comprising a container dimensioned to contain the egg yolk such that essentially only a low degree of tension is exerted on the egg yolk membrane.

Avian embryos are an interesting source for performing biological in vivo experiments, since the bird is both a vertebrate and a warm blooded animal and may therefore serve as a model organism much easier to handle than mammals. Moreover, bird embryos are cost effective and have a short developmental period (i.e. 21 days until hatch in case of a chick embryo). In addition, bird embryos are developing ex utero on the egg yolk and may easily be analyzed with optical methods.

In principle, avian embryos may be utilized for testing the effect of substances on the living organism by simply topically applying the substance on the embryo, that grows on the surface of the yolk. Such assays may, however, only be performed, when the yolk remains intact during the application and the subsequent incubation period. Primarily, the integrity of the yolk sac during the different operations is one of the main prerequisites for a successful testing.

So far, several methods for culturing avian embryos are known in the art.

In EP-B-0 295 964 a process for the in vitro culture of an avian embryo up to blastoderm formation is described, which process comprises culturing the fertilized ovum having a surrounding capsule of dense albumen partially submerged in a culture medium. The medium is selected to be essentially in line with the germinal disc.

The EP-B-0 511 431 discloses an in vitro culture method for a fertilized ovum of a hen up to blastoderm stage. The fertilized ovum is partially submerged in a container comprising thin albumen, with the yolk being covered with a sheet of gauze.

DE 586 375 discloses a device for testing eggs and for separating egg yolk from egg white, comprising a container containing though holes and a device, covering said through holes. The device essentially has the same form as the container and the same number and size of though holes as the container, and is arranged in the container such that by movement of the device in the container the through holes in the container may be opened by aligning the though holes of the device with those of the container.

Further, the DE 35 30 336 teaches a method for cultivating poultry embryos for toxicological tests. For this purpose the egg shells and shell membranes are separated from the fertilized egg. The remaining parts of the egg are placed in a container, provided with a water-vapor permeable membrane, and incubated at a predefined range of temperatures.

One of the shortcomings of the prior art methods resides, however, in that they are not appropriate for large scale processes, and in particular not suitable for processes comprising automated handling involving mechanical devices. During handling of the in vitro culture the yolk sac is subjected to physical stress, such as vibration and agitation, which is e.g. caused by movements during automated handling or occurring during application of substances onto the embryo, which often leads to a tearing of the sac.

The present invention aims to overcoming these problems and in particular to provide the possibility for a large-scale in vitro culture of avian embryos, wherein the handling of the cultures will not result in a destruction of the yolk sac.

These problems have been solved by a method for shell-less in vitro culturing of avian embryos, which comprises essentially separating egg white from egg yolk of a fertilized avian egg, introducing said egg yolk sac, optionally already including the embryo and extra-embryonic structures such as the amnion and allantois, in a yolk sac container essentially dimensioned to contain the yolk sac without substantial distortion thereof, and incubating said embryo under in vitro culture conditions.

The device, with which the present method may be carried out comprises a first and a second container, said first container 1 being dimensioned such that an egg yolk sac, when transferred into said first container 1 may essentially retain its original shape, so that the sac membrane is not subjected to an excessive stress, with the second container 2 being in contact with or adjacent to said first container 1.
In the figures,
Fig. 1 shows a sectional view of the device to be used in the present invention, comprising a first container 1 containing a yolk sac 3 and an embryo 5, a second container 2 containing egg white 4, and a cover 6; and
Fig. 2 shows a top view of the device to be used in the present invention.

In the method of the present invention, in general, the physical integrity of the egg shell is destroyed, e.g. by deleting a part of the egg shell distant from the center, in order to permit an access to, and the separation of the egg yolk from the egg white. Subsequently, egg yolk is separated partially or totally from the egg white and placed in a container dimensioned such that the yolk sac may essentially retain its original shape and form. In the following, this container will be referred to as yolk container or yolk sac container. In the context of the present application the term "essentially retaining its original form" is meant to designate a shape and form, similar to that prevailing in the egg and which does not exert a substantial stress on the yolk sac's membrane, which would occur, when the yolk sac is put on a flat surface. Typically, this is achieved by adapting the dimension of the egg yolk container to the volume of the yolk sac.

Depending on the desired application, the egg white separation is performed partially or completely. Preferably, not more than 25 %, and more preferably not more than 10 % of the total egg white of the avian egg should be present in the yolk container. Preferably, the yolk sac should be positioned in the container such that the developing embryo is on top of the yolk.

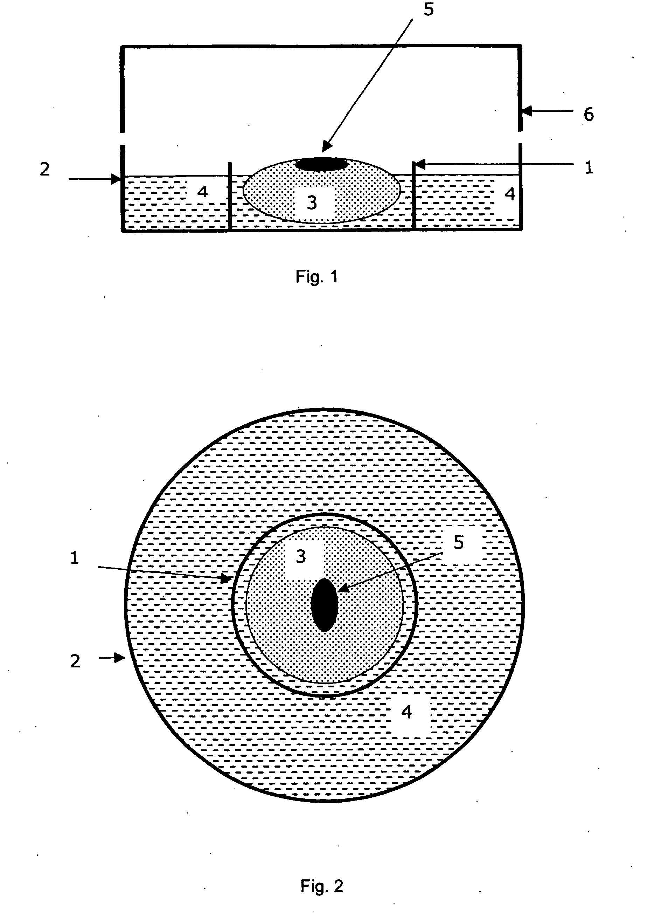

The egg white may be collected in a second, advantageously larger container, which is in direct contact with or adjacent to the yolk container, as illustrated in Figs. 1 and 2. Alternatively, the egg white may be collected in another container, e.g. in case the egg white several eggs is pooled.

Subsequently, the embryo is submitted to an incubation under conditions well known in the art. Preferably, during incubation the temperature will be set at a temperature from about 36 °C to about 39 °C, more preferably at about 37 - 38 °C, and a humidity of air of from about 40 - 95 %, 30-70%, preferably of from 40-60%, more preferably from 45 - 55 %.

Any fertilized egg collected directly or up to 10 days after lay and optionally stored at a temperature about 14-24°C, preferably about 16-19°C, preferably collected directly after lay or even a fertilized ovum obtained by surgical methods before lay may be used.

The method of the present invention is not limited to specific bird species and may be applied for an in vitro embryo culture of birds of any size. Depending on the chosen bird species, the size of yolk sac and embryo and consequently also the dimensions of the container will largely vary. Preferably, said avian embryo is a chicken (Galliformes) or quail (Turnici-formes) embryo.

According to a preferred embodiment the first and the second step of the present method may also be performed simultaneously. To this end, the entire egg may be introduced into the first container. Since the first container is dimensioned such as to receive and essentially contain only the yolk sac, said yolk sac, when getting into the first container will urge any egg white already present there out of the first container, so that substantially only the egg yolk remains in the first container. The egg white will, therefore, arrive in the second container.

Additionally and in order to alleviate processing one or both of the containers may be prefilled with water of preferably a buffer, such as e.g. phosphate buffer saline (PBS), the purpose of which is to keep an adequate humidity level in the containers and avoid a dehydration of the embryo and/or its extra-embryonic structures.

According to a preferred embodiment, the yolk sac may be sheltered during the in vitro culture with a cover, so as to avoid unwanted penetration into the embryo and thus culture with a cover, so as to avoid unwanted penetration into the embryo and thus destruction thereof during handling. The cover is advantageously removable and made of a transparent material permitting an optical analysis of the embryo developing in the yolk sac. Alternatively, any other covering material inclusive films may be chosen.

All the steps of the method of the present invention may be carried out manually or by using mechanical devices in automated processes.

The device for an in vitro culture of avian embryos comprises a first and a second container. Preferably, the said first and second container are integrally formed containers and may be e.g. in the form of compartments in a larger device.

The first container is dimensioned such that an egg yolk when placed in said first container essentially retains its original shape and/or integrity.

The second container, i.e. the container receiving the egg white, is in contact with or adjacent to said first container. Preferably, the second container is larger than the first container and completely surrounds the yolk container, which arrangement allows an easy separation of yolk sac and egg white, as exemplarily illustrated in Figs. 1 and 2. Various other arrangements are also conceivable, such as e.g. a yolk container being in contact with the second container only at one or two side(s), or a yolk container being arranged directly above the second container. The first container may be formed integrally with the second container. Likewise, the first container may be fixed by an appropriate means to the second container, which fixing means may be removed/loosened after transfer of the yolk sac to the first container.

The second container and/or the device may have any form appropriate to receive the yolk sac such that the membrane does not essentially suffer an excessive stress, e.g. an essentially parallelepiped or conical form permitting an easy storage and handling of the container(s).

For analytic purposes, e.g. by the means of automated devices using optical or acoustical methods, the second container and/or the device has/have preferably the dimensions of standard micro-reaction well-plates or standard micro-reaction recipients. If desired, a large number of devices may be combined to a large scale arrangement of relatively low height allowing a storage and handling of a great number of in vitro culture units. If desired, the first container or an arrangement of first containers may be removable from the device in order to achieve an even lower height during e.g. analytical tests.

Advantageously, one or both of the containers are provided with one or more apertures or predefined breaking points giving rise to apertures upon breaking, which apertures provide for an inlet or an outlet of fluids. This offers the possibility to remove fluids, such as e.g. egg white from the first and/or second container letting it flow through the apertures. The apertures are preferably provided in a re-sealable form, e.g. in form of a septum.

Additionally, the first and second containers may be connected via apertures or membranes permitting an exchange of the fluids present in the containers. According to a preferred embodiment the containers are completely separated.

The size of the yolk container is selected depending on the volume/size of the yolk sac and will thus vary depending on the bird species. Preferably, the yolk container may have a cylindrical form and a height of from about 0.5 cm to 6 cm and a diameter of from 1 cm to 10 cm, more preferably a height of from about 0.8 cm to 3 cm and a diameter of 3 cm to 7 cm. In general, the diameter of a cylindrical container will be chosen such that said container diameter is about 3 % to 25 %, preferably 5 % to 10 % larger than the average yolk sac diameter of the chosen bird species in the egg. However, apart from cylindrical containers also half-spherical, half-oval or parallelepiped yolk containers are within the scope of the present invention and the form of the yolk container is not to be considered as limiting the present invention. The volume of the yolk container will be selected in general in the range of 0.5 cm³ to 500 cm³, preferably from 5 cm³ to 120 cm³.

The device may be provided with a cover being e.g. useful for the humidity regulation. Preferably the cover is easily removable, in particular automatically removable. Containers 1, 2 and cover 6 may be made of one or more appropriate materials, such as e.g. glasses, metals, alloys, film materials, membranes etc.. Preferably, they will be made of plastics. Advantageously, they will be at least partially transparent in order to allow for an easy monitoring and/or analysis of in vitro culture and embryo by the means of e.g. optical devices.

The device for in vitro culturing avian embryos may be used for a large variety of toxicological, medical, pharmaceutical and chemical tests, such as e.g. large scale screening tests of toxic or pharmaceutical active substances, permitting the observation of relevant effects on one or more organs of the animal organism.

The present invention provides a largely increased physical stability of the yolk sac which also helps to increase the survival rate of chick embryos during handling. Thus, the present invention provides an in vitro culture method, which may be used on a large scale and, also performed on an automated basis.

The following examples illustrate the invention in a more detailed manner. It has, however, to be understood that the present invention is not limited to the examples but is rather embraced by the scope of the claims.

### Example 1

### Decreased rate of tearing of yolk sac during an incubation of 24 or 48 hours

Fertilized Bovans Goldline chicken eggs (12 h after laying, kept at 18°C) were incubated at 38 °C and 45 % humidity during 72 h (Motorbruter 126-EM, Bruja). The eggshell was then broken, egg shell and shell membranes were removed, and embryo, as well as yolk were placed in container 1 (petri dish, diameter: 5 cm, Greiner), while the egg white is filled in container 2 (petri dish, diameter: 9.4 cm, Greiner). About 80 % to 90 % of the total egg white were separated from yolk and embryo during these process steps. The arrangement of containers 1 and 2 is illustrated in Fig. 1 and 2. 10 mL sterile water were added to the egg white. Container 1 and container 2 were closed with a plastic lid (as illustrated in Fig. 1) and were then incubated at 38 °C and 45 % humidity.

For comparative purposes, an incubation of chick embryos without a separation of egg white from yolk and embryo was performed.

Fertilized Bovans Goldline chicken eggs (12 h after laying, kept at 18°C) were incubated at 38 °C and 45 % humidity during 72 h. The eggshell was then broken, egg shell and shell membranes were removed, and egg white, embryo, and yolk were placed in a container (petri dish, diameter: 9.4 cm, Greiner). The petri dish was covered with a corresponding lid and placed in a dish having a larger diameter (14 cm) comprising 10 ml of sterile water. The water was filled up each day. The number of in vitro cultures having a torn yolk sac was determined after 24 h and 48 h of incubation (Table 1). For the 24 h study, the respective two groups comprised 36 in vitro cultures each, and for the 48 h study 46 in vitro cultures each.

**Table 1**

| Tearing rate (indicated in percent) after 24 (n=36) and 48 (n=46) hours | | |
|---|---|---|
| | 24 h | 48 h |
| without container 1 | 34 % | 56 % |
| with container 1 | 0 % | 0 % |

These studies show that during an incubation of 24 h and 48 h a tearing of the yolk sac may be prevented by applying the device and method of the present invention.

### Example 2

### Tearing of yolk sac caused by agitation

Fertilized Bovans Goldline chicken eggs (12 h after laying, kept at 18°C) were incubated at 38 °C and 45 % humidity during 72 h. The egg shell was then broken, egg shell and shell membranes were removed, and embryo, as well as yolk were placed in container 1 (petri dish, diameter: 5 cm, Greiner), while the egg white is filled in container 2 (petri dish, diameter: 9.4 cm, Greiner). About 80 % to 90 % of the total egg white were separated from yolk and embryo during these process steps. The arrangement of container 1 and 2 is illustrated in Fig. 1 and 2. Container 1 and container 2, respectively, were closed with standard plastic lids and were shaken at 80 movements per minute by the means of a laboratory shaker (IKA Labortechnik, HS 501).

For comparative purposes, in vitro chick embryo culture arrangements (without separation of yolk/embryo and egg white) were prepared in petri dishes (diameter: 9.4 cm, Greiner), as described in Example 1, and were shaken at 80 movements per minute by means of the above mentioned shaker. The number of in vitro cultures having a torn yolk sac was determined after 10 minutes (Table 2). The respective groups comprised 5 in vitro cultures each.

**Table 2**

| Tearing rate (indicated in percent) after shaking during 10 minutes (80 movements per minute) (n=5) | |
|---|---|
| without container 1 | 80 % |
| with container 1 | 0 % |

These studies show that a tearing of the yolk sac may be completely prevented during 10 minutes of shaking at 80 movements per minute by applying the device. This test simulates the situation during e.g. various automated processes.

### Example 3

### Survival rate after 24 hours incubation

Fertilized Bovans Goldline chicken eggs (12 h after laying, kept at 18°C) were incubated at 38 °C and 45 % humidity during 72 h. The eggshell was then broken, egg shell and shell membranes were removed and embryo, as well as yolk were placed in container 1 (petri dish, diameter: 5 cm, Greiner), while the egg white is filled in container 2 (petri dish, diameter: 9.4 cm, Greiner). About 80 % to 90 % of the total egg white were separated from yolk and embryo during these process steps. The arrangement of containers 1 and 2 is illustrated in Fig. 1 and 2. 10 mL sterile water were added to the egg white. Container 1 and container 2 were closed with a standard plastic lid (as illustrated in Fig. 1) and then incubated at 38 °C and 45 % humidity.

For comparative purposes, an in vitro chick embryo culture (without separation of yolk/embryo and egg white) was prepared in a petri dish (diameter: 9.4 cm, Greiner), as described in Example 1. The petri dish was covered with the corresponding standard plastic cover and placed in a large dish (14 cm diameter) containing 10 mL of sterile water. The survival rate was determined after an incubation during 24 h under the above-mentioned conditions (Table 3). The test groups comprised 52 and 36 in vitro cultures, respectively.

**Table 3**

| Survival rate (indicated in percent) after 24 hours incubation (38 °C and 45 % humidity) | |
|---|---|
| without container 1 (n=52) | 82 % |
| with container 1 (n=36) | 94 % |

These studies show that the survival rate of embryos in in vitro cultures may be significantly increased by applying the device.

### Example 4.

### Growth velocity during an incubation during 48 hours

Fertilized Bovans Goldline chicken eggs (12 h after laying, kept at 18°C) were incubated at 38 °C and 45 % humidity during 72 h. The eggshell was then broken, egg shell and shell membranes were removed, and embryo, as well as yolk were placed in container 1 (petri dish, diameter: 5 cm, Greiner), while the egg white is filled in container 2 (petri dish, diameter: 9.4 cm, Greiner). About 80 % to 90 % of the total egg white were separated from yolk and embryo during these process steps. The arrangement of container 1 and 2 is illustrated in Fig. 1 and 2. 10 mL sterile water was added to the egg white. Container 1 and container 2 were closed with a standard plastic cover, as illustrated in Fig. 1, and the in vitro culture was incubated at 38 °C and 45 % humidity.

For comparative purposes, an in vitro chick embryo culture (without separation of yolk/embryo and egg white) was prepared in a petri dish (diameter: 9.4 cm, Greiner), as described in Example 1. The petri dish was covered with the corresponding standard plastic cover and placed in a large dish (14 cm diameter) containing 10 mL of sterile water. The embryo growth was measured after 48 hours incubation under the above-mentioned conditions on basis of the eye diameter as a marker (Binocular (Zeiss, Stemmi 2000-C) (Table 4). The respective groups comprised 7 in vitro cultures each.

**Table 4**

| Mean value of the eye diameter after 48 hours in vitro culture (n=7) (in millimeter) | |
|---|---|
| | eye diameter |
| without container 1 | 2,02 |
| with container 1 | 3,28 |

These studies show that an increased embryo growth may be observed during an incubation of 48 h by applying the device.

## Claims

1. A method for a shell-less in vitro culture of avian embryos, said method comprising the steps of:
a) essentially separating egg white of a fertilized avian egg from the yolk sac,
b) introducing the yolk sac into a yolk container (1), dimensioned such that the yolk sac essentially retains its form in said yolk container (1); and
c) incubating said embryo under in vitro culture conditions.

2. Method according to claim 1, **characterized in that** step a) and b) are performed simultaneously.

3. Method according to claim 1 to 2, comprising the step of introducing said egg white in a second container (2), said second container being in contact with or adjacent to said yolk container (1) and optionally comprising the step of adding water or an aqueous buffer system to said egg white.

4. Method according to claim 1 to 3, comprising the step of covering said the yolk sac with a cover.

5. Use of a device comprising:
a first and a second container;
said first container (1) being dimensioned such that an egg yolk when placed in said first container (1) essentially retains its original shape; and
said second container (2) being in contact with or adjacent to said first container (1), in the method for in vitro culture of avian embryos as defined in anyone of claims 1 to 4, in particular for toxicological, medical, pharmaceutical and chemical tests and assays.

6. The use according to claim 5, wherein said device and/or said second container (2) has an essentially parallelepipedical, cylindric or conical form.

7. The use according to claim 5, wherein said device or said second container (2) has the outer dimensions of standard micro-reaction well-plates or standard micro-reaction recipients.

8. The use according to any of the claims 5 to 7, wherein one or both of said containers (1, 2) are provided with one or more apertures or predefined breaking points, which apertures or predefined breaking points are providing for an inlet or an outlet of fluids.

9. The use according to any of the claims 5 to 8, wherein that said yolk container (1) has a volume of 0.5 cm³ to 500 cm³, preferably from 5 cm³ to 120 cm³.

## Patentansprüche

1. Verfahren zur schalenlosen *in vitro* Züchtung von Vogelembryonen, welches die Schritte umfasst:
a) im wesentlichen Abtrennen des Eiweißes des befruchteten Eis vom Dottersack,
b) Einbringen des Dottersackes in einen Dotterbehälter (1), der derart bemessen ist, dass der Dottersack seine Form in dem Dotterbehälter (1) im wesentlichen beibehält; und
c) Inkubieren des Embryos unter *in vitro* Bedingungen.

2. Verfahren nach Anspruch 1, **dadurch gekennzeichnet, dass** Schritt a) und b) gleichzeitig ausgeführt wird.

3. Verfahren nach Anspruch 1 bis 2, welches die Schritte umfasst, Einbringen des Eiweisses in einen zweiten Behälter (2), wobei der Behälter mit dem Dottersackbehälter (1) in Kontakt steht oder an diesen angrenzt, und wahlweise den Schritt umfasst, dem Eiweiß Wasser oder eine wässrige Pufferlösung hinzuzufügen.

4. Verfahren nach Anspruch 1 bis 3, welches den Schritt umfasst den Dottersack mit einer Abdeckung abzudecken.

5. Verwendung einer Einrichtung umfassend:
einen ersten und einen zweiten Behälter,
wobei der erste Behälter (1) derart bemessen ist, dass ein Eidotter im wesentlichen seine ursprüngliche Form behält wenn er in den ersten Behälter (1) eingebracht wird; und
wobei der zweite Behälter (2), mit dem ersten Behälter (1) in Kontakt steht oder an diesen angrenzt,
in dem Verfahren zur *in vitro* Züchtung von Vogelembryonen nach einem der Ansprüche 1 bis 4, insbesondere fiir toxikologische, medizinische, pharmazeutische und chemische Tests und Assays.

6. Verwendung nach Anspruch 5, wobei die Einrichtung und/oder der zweite Behälter (2) eine im wesentlichen parallelepipedische, zylindrische oder konische Form hat.

7. Verwendung nach Anspruch 5, wobei die Einrichtung und/oder der zweite Behälter (2) die äußeren Abmessungen von Standard-Mikrotiterplatten oder Standard-Mikroreaktionsgefäßen hat.

8. Verwendung nach einem der Ansprüche 5 bis 7, wobei einer oder beide Behälter (1, 2) mit einem oder mehreren Öffnungen oder Sollbruchstellen ausgestattet sind, wobei die Öffnungen oder Sollbruchstellen ein Einströmen und Ausströmen von Fluiden ermöglichen.

9. Verwendung nach einem der Ansprüche 5 bis 8, wobei der Dotterbehälter (1) ein Volumen von 0,5 cm³ bis 500 cm³, vorzugsweise von 5 cm³ bis 120 cm³ hat.

## Revendications

1. Méthode pour cultiver *in-vitro* des embryons d'oiseau hors de la coquille de l'oeuf, ladite méthode comprenant les étapes de :
a) séparation essentielle du blanc d'un oeuf d'oiseau fertilisé de la poche nutritive,
b) introduction de la poche nutritive dans un récipient pour le jaune (1), dimensionné de telle manière qu'il permette à la poche nutritive de conserver essentiellement sa forme dans le récipient pour le jaune (1) ; et
c) incubation dudit embryon dans des conditions de culture *in-vitro.*

2. Méthode selon la revendication 1, **caractérisée par le fait que** les étapes a) et b) sont réalisées simultanément.

3. Méthode selon les revendications 1 à 2, comprenant l'étape d'introduction dudit blanc d'oeuf dans un second récipient (2), ledit second récipient étant en contact avec ou adjacent avec ledit récipient pour le jaune (1) et comprenant éventuellement l'étape d'addition d'eau ou d'un système de tampon aqueux dans ledit blanc d'oeuf.

4. Méthode selon les revendications 1 à 3, comprenant l'étape de recouvrement de la poche nutritive avec un couvercle.

5. Utilisation d'un dispositif comprenant :
un premier et un second récipient ;
ledit premier récipient (1) étant dimensionné de telle manière qu'un jaune d'oeuf placé dans ledit premier récipient (1) retienne essentiellement sa forme originale; et
ledit second récipient (2) se trouvant en contact avec ou adjacent avec ledit premier récipient (1), dans la méthode de culture *in-vitro* d'embryons d'oiseaux selon l'une quelconque des revendications 1 à 4, en particulier pour des tests et dosages toxicologiques, médicaux, pharmaceutiques et chimiques.

6. Utilisation selon la revendication 5, lorsque ledit dispositif et/ou ledit second récipient (2) a une forme essentiellement parallélépipédique, cylindrique ou conique.

7. Utilisation selon la revendication 5, lorsque ledit dispositif ou ledit second récipient (2) a les dimensions extérieures d'une plaque standard à puits de micro-réaction ou de récipients standard de micro-réaction.

8. Utilisation selon l'une des revendications 5 à 7, lorsqu'un ou les deux dits récipients (1, 2) sont équipés avec un ou plusieurs orifices ou points de cassure prédéfinis, lesquels orifices ou points de cassure prédéfinis mettant à disposition une entrée ou sortie de fluides.

9. Utilisation selon l'une des revendications 5 à 8, lorsque ledit récipient pour le jaune (1) a un volume de 0,5 cm³ à 500 cm³, préférablement de 5 cm³ à 120 cm³.
